# EUROPEAN PATENT APPLICATION

(11) **EP 1 918 365 A1**
(43) Date of publication of application: **07.05.2008**
(21) Application number: 06123181.7
(22) Date of filing: 30.10.2006
(51) Int. Cl.: C12N 5/08, C12Q 1/02

(54) **Labeling of human epidermal stem cells**

(71) Applicant: DKFZ Deutsches Krebsforschungszentrum, 69120 Heidelberg (DE); Landesstiftung Baden- Württemberg GmbH, 70191 Stuttgart (DE)
(72) Inventor: Boukamp, Petra, 64646 Heppenheim (DE); Stark, Hans-Jürgen, 74909 Meckesheim (DE); Muffler, Sonja, 68259 Mannheim (DE); Amoros-Alonso, Mara, 69120 Heidelberg (DE); Fusenig, Norbert, 69118 Heidelberg (DE)
(74) Representative: Dick, Alexander

(57) **Abstract**

The present invention provides skin equivalents comprising human epidermal stem cells which are specifically labeled. The labeling is carried out with a marker capable of labeling slowly proliferating cells, e.g. iododeoxyuridine or PKH26. Particularly, the invention provides skin equivalents comprising labeled epidermal stem cells. The invention also provides corresponding uses and methods of using such cultures, e.g. in the fields of research and medical treatment of skin diseases.

## Description

The present invention relates to labeling of human epidermal stem cells.

The human skin does not only protect the human body from the external world, but it is also a target of many kinds of infectious and non-infectious diseases, such as skin cancer, wounds or acne.

The skin is composed of the so-called epidermis, an external epithelial component, and the so-called dermis, the underlying connective tissue component. The epidermis itself is primarily composed of keratinocytes which are arranged in stratified layers. The so-called stratum basale at the dermal-epidermal junction is a single layer of keratinocytes with a small number of interspersed melanocytes. The stratum basale is also known to the person skilled in the art as stratum germinativum since it is the site of the generation of new keratinocytes by cell proliferation. The stratum germinativum is also the site where the epidermal stem cells are presumably located.

Due to its importance in disease, the study of the skin and, particularly, the epidermis has received considerable interest. However, in spite of extensive studies, testing of therapies for the treatment of skin diseases is currently cumbersome and expensive.

As mentioned, particularly the epidermis has attracted a certain interest. The epidermis is undergoing a continuous differentiation process, in which small populations of stem cells proliferate and differentiate, which change their shape and composition and are finally shed from the outer surface of the epidermis. It has recently been realized that the renewal and proliferation is provided by a very small population of stem cells (2 to 5 % or less of all basal cells). With the currently available biochemical or molecular markers it is not possible to identify human epidermal stem cells unequivocally. Instead, the available markers only recognize certain cell populations which may overlap with the human epidermal stem cell population, but are not identical with that population. For example, such markers recognize a plurality or at least groups of cells, whereas from functional studies it is currently concluded that the stem cells are distributed evenly in the epidermis as single cells.

Epidermal stem cells are thought to be characterized by biomarkers, preferably, by high use pression of β₁,α6 integrins, or low use pression of MCSP (Melanoma chondritrinsulfat protreoglycan) CD71 transferin receptor.

Moreover, in mice, it has been possible to label a population of supposed epidermal stem cells by administration of bromodeoxyuridine (Bickenbach, J.R. (2005). Isolation, characterization, and culture of epidermal stem cells. Methods Mol. Biol., vol. 289, pp. 97-102, 2005).

However, it is well-known, that the epidermis of most laboratory animals (such as mice) is quite different from the epidermis of human subjects. Therefore, animal models are only of limited use for studying the situation in humans. In particular, in humans in vivo labeling does not to appear to be a valid option, as it may be associated with a risk of the human subject. E. g. bromodeoxyuridine is considered to be a mutagen.

Recently, an in vivo type dermal equivalents has been developed (Stark, H.J., Willhauck, M.J., Mirancea, N., et al. (2004). Authentic fibroblast matrix in dermal equivalents normalises epidermal histogenesis and dermo-epidermal junction in organotypic co-culture, Eur J Cell Biol, vol. 83, 631-645). Cells in this culture have been labeled with BrdU for 1-x h. to measure replicating cells.

There is a need for means and methods which allow studying epidermal development and the role of stem cells in human diseases as well as the response of an in vitro skin model to exogenous stimuli such as compounds to be used as cosmetic or pharmaceutical compositions. The technical problem, thus, may be seen as the provision of such means and methods. The technical problem is solved by the embodiments characterized in the claims and herein below.

The problem is solved by a human epidermal stem cell labeled with a marker capable of specifically labeling slowly proliferating cells. Preferably, said human epidermal stem cell is derived from keratinocytes isolated from their natural environment in the human skin.

The problem is also solved by an epidermal culture comprising a human epidermal stem cell, wherein the human epidermal stem cell is specifically labeled with a marker capable of specifically labeling slowly proliferating cells.

The epidermal culture referred to herein above, preferably, resembles a skin equivalent as described in detail elsewhere in this specification.

The invention is based on the finding that an epidermal culture according to the invention contains stem cells which are quite similar to the in vivo situation. This finding was rather unexpected as the culture and maintenance of stem cells in vitro is notoriously difficult. The finding is particularly surprising in the case of those epidermal cultures which are derived from isolated keratinocytes seeded on to a matrix.

However, apparently a level of tissue homeostasis can be reached which allows the maintenance of epidermal stem cells. In particular, it has been found in the context of the invention that the epidermal stem cells in the culture are maintained as slowly proliferating cells and that it is possible to label these cells with a marker capable of labeling slowly proliferating cells. Furthermore, it has been found in a context of the invention that there appear to be no other slowly proliferating cells in the culture. Thus, it is possible to label the stem cells in the culture rather specifically with a marker capable of specifically labeling slowly proliferating cells.

Unless otherwise specified, the methods of cell and molecular biology referred to in this specification (e.g. manipulations of nucleic acids, peptides, polypeptides or proteins) can be performed using standard methods of molecular biology and immunology (see, e.g. Maniatis et al. (1989), Molecular cloning: A laboratory manual, Cold Spring Harbor Lab., Cold Spring Harbor, NY; Ausubel, F.M. et al. (eds.) "Current protocols in Molecular Biology". John Wiley and Sons, 1995; Tijssen, P., Practice and Theory of Enzyme Immunoassays, Elsevier Press, Amsterdam, Oxford, New York, 1985).

The term "epidermis" is known to the person skilled in the art. The epidermis is the outer part of the skin. The skin itself is composed of the epidermis and the so-called dermis, which is the underlying connective tissue component. The epidermis is an external epithelial component, which is primarily composed of keratinocytes, which are arranged in stratified layers.

The terms "epidermal stem cell" and "stem cell" are known to the person skilled in the art. More particularly, the term "stem cell" relates to an undifferentiated cell which can undergo unlimited division and can give rise to one or several different cell types. More particularly, it relates to a largely undifferentiated cell from which a renewable tissue, such as the skin, can be formed.

The term "epidermal stem cell", thus, more particularly relates to a stem cell which can give rise to keratinocytes of all stages present in the epidermis.

The invention takes advantage of certain markers. The term "marker" is known to the person skilled in the art. A suitable marker according to the invention is an entity, which is capable of specifically labeling slowly proliferating cells, more particularly, an entity which does specifically label slowly proliferating cells. The term "specifically" in this context is understood by the person skilled in the art. Preferably, the term relates to predominantly labeling slowly proliferating cells. More preferably, the term relates to labeling slowly proliferating cells with at least two times, more preferably at least 3, 5, 10, 20, 50, or most preferably at least 100 times the intensity as compared to a relevant other substrate (e.g. a rapidly proliferating cell, particularly a rapidly proliferating cell present in the same culture or under the same cultivation and/or labeling conditions). Suitable markers are known in the art and specific examples are given in this description.

In the context of the invention, the chemical nature of the marker is of little importance. For example, the marker can be of high or low molecular weight, it can be an organic or non-organic compound, a peptide, a polypeptide or protein, an antibody or aptamer, a nucleic acid, a lipid, or any other kind of chemical compound fulfilling the functional requirements as laid out in this specification.

The term "antibody" as used herein includes both polyclonal and monoclonal antibodies, as well as variants or fragments thereof, such as Fv, Fab and F(ab)₂ fragments that are capable of binding antigen or hapten. The term "antibody" also includes single-chain antibodies. Preferably, the antibody binds with a dissociation constant Kd of 10⁻⁷ M or lower, more preferably with a Kd of 10⁻⁸ M or lower, more preferably with a Kd of 10⁻⁹ M or lower, most preferably with a Kd of 10⁻¹¹ M or lower.

Preferably, the marker is capable of specifically binding to slowly proliferating cells or is capable of becoming enriched in slowly proliferating cells (e.g. during culture of the cell or a culture comprising the cell). For example, the marker may be incorporated into the cell, e.g. into the DNA or membranes. Thus, the marker will be distributed onto any daughter cells derived from the cell loaded with the maker. Over the course of a following cultivation, the marker will be more strongly diluted in cells which proliferate rapidly, and the marker will be less diluted in cells which proliferate more slowly. Thus, the marker will become relatively enriched in more slowly proliferating cells as compared to more rapidly proliferating cells.

The term "specifically binding" is understood by the person skilled in the art. Specific binding preferably relates to binding with at least two times, more preferably at least 3, 5, 10, 20, 50, or most preferably at least 100 times the affinity to the target (e.g. a slowly proliferating cell) as compared to a relevant other substrate (e.g. a rapidly proliferating cell).

According to the invention, the marker is capable of specifically "labeling" slowly proliferating cells, more particularly, the marker specifically labels slowly proliferating cells. The term "labeling" in this context is known to the person skilled in the art. More particularly, the term "labeling" relates to making the thus labeled cells detectable and/or traceable. More particularly, the term "labeling relates to making the thus labeled cell distinguishable from other cells (e.g. other cells present in an epidermal culture). Preferably, the label is capable of making the labeled cell detectable or distinguishable within the context of a culture, e.g. to make the cell specifically identifiable within a live culture or on histological sections of a tissue culture.

Therefore, the marker may be comprise, consist of, or be coupled covalently or non-covalently to a label allowing detection of the marker, preferably in a semi-quantitative or quantitative manner. Also the term "label" in this context is known to the person skilled in the art.

Labeling of the marker may be carried out by direct or indirect methods and it may be carried out before or after contacting the cell with the marker. Direct labeling involves coupling of the label directly (covalently or non-covalently) to the marker. Indirect labeling involves binding (covalently or non-covalently) of a ligand to the marker. The ligand should specifically bind to the marker. Said ligand may be coupled with a suitable label and/or be the target (receptor) of secondary ligand binding to the first ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.)

Preferred ligands include antibodies, nucleic acids, peptides or polypeptides, and aptamers, e.g. nucleic acid or peptide aptamers (e.g. spiegelmers or anticalins). Methods to obtain such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display.

Regarding the terms "antibody" and "specifically binding" as used herein it is referred to the above definitions.

Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels.

The labeling also relates to "tagging" the marker (or a respective ligand) with one or more tags as known in the art. Such tags may then be targets for higher order ligands, which then may carry a suitable label. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus.

Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). Particularly, localized deposition of an enzymatic reaction product in the proximity of the cell will make a cell identifiable within the context of a culture.

For measurement of enzymatic reaction products, preferably the amount of substrate is saturating. Preferably, the sample is contacted with the substrate for an adequate period of time. An adequate period of time refers to the time necessary for an detectable, preferably measurable amount of product to be produced. Instead of measuring the amount of product, the time necessary for appearance of a given (e.g. detectable) amount of product can be measured.

Typical fluorescent labels include fluorescent proteins (such as GFP, YFP, RFP and derivatives thereof), Cy3, Cy5, Texas Red, Fluorescein, the Alexa dyes (e.g. Alexa 568), and quantum dots. Further fluorescent labels are available e.g. from Molcular Probes (Oregon).

Typical radioactive labels include ³⁵S, ¹²⁵I, ³²P, ³³P, ³H and the like. A radioactive label can be detected by any method known and appropriate, e.g. a light-sensitive film or a phosphor imager.

Examples for suitable markers comprising a label include thymidine, which can be labeled by incorporation of ³H, and the compound PKH26 (Sigma, St. Louis, MO, USA), which is detectable by its fluorescent properties.

Examples for markers detectable by suitable ligands include iododeoxyuridine and bromodeoxyuridine (e.g. detectable with suitable antibodies).

Preferably, the marker is a non-toxic marker and/or a non-mutagenic marker and/or a marker which can be detected in the living cell. However, an advantage of the present invention is that also a mutagenic and/or slightly toxic markers can be used, as the labeling is performed in vitro. In fact, a mutagenic or a toxic marker may be advantageous for superior quality of the label or for ablating the labeled cell.

Therefore, the present invention also relates to a method of ablating epidermal stem cells labeled with a marker according to the present invention, particularly in an epidermal culture.

An advantage of a marker which can be detected in the living cell is that is allows to track the cell and to observe the behaviour of the cell without having to destroy the cell or the epidermal culture. Thus, it is also possible to observe the behaviour of the cell in response to a particular cosmetic or therapeutic treatment regimen.

Labeling of epidermal stem cells allows it to further investigate or isolate the epidermal stem cells (e.g. to isolate them from an epidermal culture by dissociating the culture and sorting the cells, e.g. according to the presence of a fluorescent label (e.g. by fluorescence-activated cell sorting, FACS).

According to the invention, the amount of marker is sufficient to label the cell sufficiently to make it detectable and/or traceable. Such amounts are known to the person skilled in the art and depend on the choice of a suitable detection system as well as the choice of a suitable amount of marker, which is well within the skill of the person skilled in the art.

According to the invention, the marker is capable of specifically labeling slowly proliferating cells. The person skilled in the art is able to determine which markers fulfil this requirement. Furthermore, such markers are commercially available and the examples are given further below. A suitable assay to determine whether a marker is capable of specifically labeling slowly proliferating cells may include a cell culture made up of slowly proliferating and rapidly proliferating cells and labeling these cells with the candidate marker. A marker to be used as a label in accordance with the present invention shall exclusively label the slowly proliferating cells in the aforementioned co-culture setup. More particularly, an epidermal culture or a dermal equivalent as further described below, particularly in Examples 3 and 4, can be used for such an assay. If the candidate for the marker labels the same cell population or a largely overlapping cell population with the cell population labeled with iododeoxyuridine, then the marker is particularly suited in the context of the present invention. An example for a labeling protocol with iododeoxyuridine in a dermal equivalent is given further below (Example 6). Preferably, the candidate for a marker is capable of specifically labelling the same cell population under the conditions laid out in said example.

The terms "slowly" proliferating and "rapidly" proliferating cells are well understood by the person skilled in the art. Specifically, a slowly proliferating cell in accordance with the present invention is capable of retaining the label specified elsewhere in this description for at least two, at least three or at least four weeks. The term "slowly" proliferating may also relate to a non-proliferating cell, but preferably to cells which show at least some proliferation and/or are not terminally differentiated. More particularly, the term "slowly proliferating" relates to a cell proliferating with the same or approximately the same rate as the cells labeled with iododeoxyuridine as described in Example 6 or with a rate of division which is not more than 4 times as high, more preferably not more than two times as high, more preferably, not more than 1.5 times as high as the average division rate of the cells labeled with iododeoxyuridine as given in Example 6.

The term "specifically" in the context of labeling slowly proliferating cells is well understood by the person skilled in the art. More particularly, the term "specifically" relates to predominantly or exclusively labeling slowly proliferating cells as compared to rapidly proliferating cells. A certain degree of labeling of other cells is well acceptable, if the slowly proliferating cells can be detected, traced or distinguished from such other cells, e.g. by a different degree of intensity of labelling or by a different location. More preferably, the marker is capable of labeling at least 50 %, more preferably at least 60 %, 70 %, 80 %, 90 %, most preferably at least 95 % of the slowly proliferating cells, most preferably of the cells labeled with iododeoxyuridine in an epidermal culture according to Example 6. Furthermore, the marker does not label more than 30 %, 25 %, 20 %, 15 %, 10 %, most preferably, not more than 5 % of any quickly proliferating cells in the same sample, i.e. most particularly, the cells which are apparently not labeled with iododeoxyuridine in the epidermal culture according to Example 6.

Examples for suitable markers include, but are not limited to: Agents (particularly nucleotide analogues) which are incorporated into a cell's DNA during replication (e.g. iododeoxuridine), fluorescent molecules which are incorporated into the cell membrane (e.g. PKH26 (labeling kit sigma, St. Louis, MO, USA) and Quantin dots). Preferred fluorescent molecules are those provided by Sigma as MINI26, PKH26-PCL, PKH67, MINI67, PKH67-PCL, PKH26, PKH26-PCL.

In a particular embodiment, the present invention relates to an epidermal culture comprising human epidermal stem cells which are specifically labeled with a marker capable of specifically labeling slowly proliferating cells. More preferably, said epidermal culture comprises a stem cell population specifically labeled with a marker capable of specifically labeling slowly proliferating cells. Advantageously, the invention thus provides an epidermal culture comprising labeled human epidermal stem cells. As already mentioned, previously the labeling of human epidermal stem cells has not been possible in a satisfying manner, more particularly, it has not been possible to specifically label stem cells in an epidermal culture. E.g. it was difficult to determine whether such cultures do comprise epidermal stem cells at all. Furthermore, previous labeling methods typically labeled populations of cells which may overlap with a stem cell population present in the culture, but would not specifically label the epidermal stem cell population. The present invention provides an efficient, reliable, and inexpensive method to label such stem cells.

This is also of advantage and importance because epidermal cultures provide an important system to study the human epidermis and its diseases and treatment regimens, cosmetic or non-cosmetic as well as a scientific study object. Moreover, animal testing can be avoided in said fields thanks to the method of the present invention.

The term "epidermal culture" is well understood by the person skilled in the art. Particularly, the term comprises tissue cultures of skin or epidermal biopsies, so-called organotypic cultures according to the definitions given further below.

More particularly, the present invention relates to an epidermal culture comprising human epidermal stem cells which are specifically labeled with a marker capable of specifically labeling slowly proliferating cells, wherein at least 60 %, 70 %, 80 %, 90 %, most preferably, at least 95 % of the stem cells present in the culture are specifically labeled with a marker according to the present invention, i.e. particularly with a marker capable of specifically labeling slowly proliferating cells.

The term "specifically" in the context of labeling of human epidermal stem cells in such culture is well understood by the person skilled in the art. More particularly, the term may relate to a culture in which human epidermal stem cells are detectable and/or traceable within the epidermal culture, particularly in which human epidermal stem cells are distinguishable from other cells. More particularly the term relates to a culture in which almost exclusively epidermal stem cells are labeled, e.g. in which not more than 10%, particularly not more than 5%, more particularly not more than 4%, 3%, 2%, 1%, or 0.5% of all labeled cells are not human epidermal stem cells. A certain degree of unspecific labeling may be acceptable if such other cells can be distinguished from the epidermal stem cells by other simple means, e.g. according to their position in the culture. E.g. a differentiated keratinocyte in the upper layer may be labeled as long as it is found in a stratum of the epidermal culture in which no human epidermal stem cells are present (e.g. in the stratum spinosum, stratum granulosum)

Even more particularly, the term "specifically labeling" relates to making an epidermal stem cell population detectable and/or traceable within the epidermal culture. More particularly the term may relates to a culture in which almost all human epidermal stem cells are distinguishable from other cells. More particularly, the term may relate to a culture in which at least 60 %, more preferably at least 70 %, 80 %, 90 %, most preferably at least 95 % of the epidermal stem cells present in the epidermal culture are labeled.

The person skilled in the art is able to determine conditions suitable for such specific labeling, e.g. by comparing the results achieved with a candidate for such a specific labeling method with the population of cells labeled in the epidermal culture according to Example 6.

Preferably, the epidermal culture is an organotypic culture. The term "organotypic culture" or also sometimes termed raft cultures is known to the person skilled in the art. Organotypic cultures are culture in which the cells show organized growth in a form resembling a tissue. Examples for organotypic cultures according to the invention are provided in Examples 1 to 4. Advantageously, the labeling method according to the present invention is capable of labeling epidermal stem cells and/or the epidermal stem cell populations present in such organotypic cultures. As already mentioned, it was rather unexpected, that stem cells are present and can remain in organotypic cultures, particularly, as such cultures are not derived from skin biopsies, but from dissociated or partially dissociated cells seeded into culture, e.g. onto a matrix. It was unexpected to find epidermal stem cells in such cultures, as this would involve either dedifferentiation or maintenance of stem cells in conventional cultures of the cells from which the culture has been derived.

Furthermore, quite unexpectedly, the stem cells in such artificial system appear to be slowly proliferating, which allows to specifically label them with a marker capable of specifically labeling slowly proliferating cells. Such organotypic cultures are of particular importance in medicine and in the cosmetics industry. The advantage of using organotypic cultures instead of cultures derived from biopsies is that they can be derived easily from dissociated cells and can be moulded onto a matrix or matrices suitable for medical applications including tissue engineering or avoidance of animal testing. Therefore, it is of importance to study the stem cell behaviour in such cultures, e.g. in order to improve the methods for providing such organotypic cultures.

The organotypic culture may be a heterologous or homologous culture. Both terms are familiar to the person skilled in the art. Heterologous cultures comprise cells derived from a different species than the other cells which constitute the remaining culture.

An advantage of heterologous cultures is that cells may be used which have been obtained from genetically modified animals (e.g. knock-out fibroblasts which are already available, e.g. as genetically modified mouse fibroblasts. The application of knock-out fibroblasts changes the fibroblast-derived cytokine profile which then influences keratinocyte behaviour in the culture). Furthermore, heterologous cells added to the culture can be more easily distinguished from the other cells. The same is true for any factors secreted by such cells.

In contrast, an advantage of homologous cultures is that there is less risk of transferring animal diseases (particularly viruses) into the culture. This may be important in some applications, e.g. if the culture is to be used as a transplant in humans. In the case of a transplant for use in humans (e.g. in wound treatment), it may be preferred to use only cells of the same patient (autologous cells).

Preferably, the organotypic culture is a skin equivalent comprising a dermal portion and an epidermal portion. The term "dermal portion" means that the organotypic culture does not only resemble a tissue, but subsequently allows to develop a tissue which strongly resembles a normal human epidermis. Specifically, an epidermal portion can be developed by seeding keratinocytes onto the dermal portion of the skin equivalent.

A dermal portion can be obtained as described for dermal equivalents in the prior art. Dermal equivalents according to the invention are known in the art and have been described e.g. in Stark et al., 2004 (Stark, H.-J., Willhauck, M.J., Mirancea, N., et al. (2004). Authentic fibroblast matrix in dermal equivalents normalized epidermal histogenesis and dermo-epidermal junction in organotypic co-culture. European Journal of Cell Biology, vol. 83, pp. 631-645). Examples for such dermal equivalents are also given in Examples 1 to 4.

A dermal equivalent can e.g. be prepared based on type I collagen hydrogels. A suitable method may comprise the steps of providing a type I collagen solution, adding fibroblasts to the solution, gelling the collagen solution comprising the fibroblasts, optionally on a suitable membrane. In order to provide a skin equivalent, the method comprises the additional step of adding keratinocytes and cultivating the culture in a suitable medium. Preferably, said suitable medium comprises FBS (fetal bovine serum) and/or a corticosteroid (e.g. hydrocortisone) and/or L-ascorbic acid.

More preferably, a "skin equivalent" according to the present invention is a culture which allows to develop a complete epidermis and remains vital and proliferatively active for at least 3, preferably at least 4, 6, 8, most preferably at least 12 weeks. Such "long-term skin equivalents" appear to provide a tissue homeostasis which is particularly interesting in the study of long-term therapeutic or cosmetic regimens. Surprisingly, it has been observed by the inventors that even such long-term skin equivalents comprise a stem cell population, which can be further studied with the labeling method according to the invention. Surprisingly, the stem cell population in such culture appears to resemble the stem cell population in vivo and, thus, is of particular interest e.g. in the study of cosmetic or therapeutic treatment regimens.

Preferably, a "long-term skin equivalent" according to the invention is able to provide for at least one, preferably at least 2, more preferably at least 3 of the following functional properties in the corresponding skin equivalent: (a) co-expression of keratins K1/K10 in the corresponding skin equivalent, (b) downregulation of keratin K16, (c) restriction of integrin and K15 distribution to the basal layer, (d) restriction of keratinocyte proliferation to the basal layer, (e) de novo reconstruction of genuine dermal tissue. Said functional properties have been described in detail and can be measured according to the methods described in Stark et al., 2004 (Stark, H.-J., Willhauck, M.J., Mirancea, N., et al. (2004). Authentic fibroblast matrix in dermal equivalents normalized epidermal histogenesis and dermo-epidermal junction in organotypic co-culture. European Journal of Cell Biology, vol. 83, pp. 631-645).

Preferably, a "long-term skin equivalent" according to the invention has properties comparable to the dermal equivalent disclosed in Example 3 and/or Example 4. More preferably, a dermal equivalent according to the invention has properties comparable to the hygraft dermal equivalent disclosed in Stark et al., 2004 (Stark, H.-J., Willhauck, M.J., Mirancea, N., et al. (2004). Authentic fibroblast matrix in dermal equivalents normalized epidermal histogenesis and dermo-epidermal junction in organotypic co-culture. European Journal of Cell Biology, vol. 83, pp. 631-645).

Preferably, the "long-term skin equivalent" comprises a scaffold of fibrous material. Said fibrous material, more preferably, contains esterified higher hyaluronic acid.

A "long-term skin equivalent" can e.g. be prepared based on a suitable scaffold. A suitable method may comprise the steps of (1) providing a suitable scaffold, optionally provided on a suitable membrane, (2) introducing fibroblasts into the scaffold, (3) optionally cultivating the scaffold comprising the fibroblasts in a suitable medium. In order to provide a skin equivalent, the method shall comprise the additional step (4) of adding keratinocytes and cultivating the culture in a suitable medium.

Preferably, the fibroblast are introduced into the scaffold as a fibrin gel suspension, particularly using a fibrin glue. The term "fibrin glue" is known to the person skilled in the art. Fibrin glues are well-known due to their important medical applications (e.g. to stop internal bleedings). Fibrin glues comprise fibrinogen which is transformed into a fibrin meshwork e.g. by adding a suitable activator or enzyme. More particularly, the fibrin glue may be a two-component fibrin glue, e.g. it may comprise fibrinogen in a first component and thrombin in the second component (e.g. Baxter-Tissucol/Tisseel, a two-component fibrin glue for surgical application (Baxter, Heidelberg, Germany). Preferably, the fibrin glue is provided in a concentration allowing the formation of a gel (preferably the formation of a gel after addition of the glue to a scaffold as herein described) within a time-period of more than 1 but less than 60 minutes, more preferably of more than 2 and less than 30 minutes, more preferably of more than 3 and less than 20 minutes, more preferably of more than 5 and less than 15 minutes, most preferably of more than 8 and less than 12 minutes, at a temperature of 37 °C. Preferably, the fibrinogen is used at a concentration of 1 to 50 mg/ml, more preferably 2 to 30 mg/ml, more preferably 4 to 20 mg/ml, more preferably 6 to 15 mg/ml, most preferably at approximately 8 mg/ml. If the fibrinogen component is diluted, the diluting is preferably carried out in absence of Ca²⁺ and Mg²⁺, e.g. in PBS (phosphate buffered saline) without Ca²⁻ and Mg²⁺, preferably at approximately neutral pH, e.g. pH 7.0. The activator component is preferably used at a fibrinogenic activity corresponding to a thrombin concentration of 1 to 100 units, more preferably 2 to 50 units, more preferably 5 to 20 units, more preferably 8 to 15 units, most preferably approximately 10 units. E.g. a thrombin component may be diluted to said concentrations using PBS as diluent. Preferably, the fibroblasts are added to the scaffold in the a solution comprising the activator (e.g. thrombin) component but not the fibrinogen component. The fibrinogen component is then added in a second step to the scaffold with the fibroblasts, so that a clot enclosing the fibroblast is formed.

Preferably, said suitable medium comprises FBS (fetal bovine serum) and/or L-ascorbic acid and/or an agent with an activity corresponding to TGFβ1. More preferably, the medium is based on Dulbecco's modified Eagle's Medium (DMEM). Preferably the FBS is at a concentration of 5 to 20%, more preferably 7 to 15%, more preferably 8 to 12%, most preferably approximately 10%. Preferably the L-ascorbic acid is at a concentration of 5 to 500 µg/ml, more preferably 10 to 200 µg/ml, more preferably 20 to 100 µg/ml, more preferably 30 to 80 µg/ml, most preferably approximately 50 µg/ml. Preferably the agent with the activity corresponding to TGFβ1 is at a concentration corresponding to 0.2 to 5 ng/ml, more preferably 0.3 to 3 ng/ml, more preferably 0.5 to 2 ng/ml, more preferably 0.8 to 1.5 ng/ml, most preferably approximately 1 ng/ml of recombinant human TGFβ1.

Preferably, the agent with an activity corresponding to TGFβ1 is TGFβ1, more preferably recombinant human TGFβ1.

Preferably, the keratinocytes are seeded on top of the scaffolds with the fibroblasts. More preferably, the keratinocytes are added after pre-cultivation of the scaffolds in said suitable medium (e.g. for 0 to 7 days, preferably for approximately 1 to 3 days).

Preferably, the fibrin meshwork in the scaffolds with the fibroblasts is largely maintained after adding the keratinocytes. This can be achieved e.g. by adding a suitable fibrinolysis inhibitor, e.g. aprotinin. E.g. the medium may be supplemented with aprotinin shortly before adding keratinocytes to the culture and/or during subsequent cultutivation. Preferably, the fibrinolysis inhibitor is at a concentration corresponding to the fibrinolysis inhibiting activity of 50 to 2000, preferably 100 to 1500, more preferably 100 to 1000 units per ml, more preferably 150 to 700, more preferably 150 to 400, most preferably approximately 200 units per ml of aprotinin. The concentration may be approximately three times or two times as high in the medium which is used immediately before adding the keratinocytes.

In another embodiment, the invention relates to a method of labeling an epidermal stem cell, comprising the steps of
a) providing an epidermal stem cell or an epidermal culture (particularly an organotypic culture, more particularly a skin equivalent) or a keratinocyte,
b) contacting the culture or keratinocyte with a marker according to the invention, particularly a marker capable of specifically labeling slowly dividing cells,
c) optionally, washing the culture in order to remove excess label,
d) optionally, repeating steps b) and c),
e) further cultivating the cell or culture, preferably for at least 2 days, more preferably at least 4 days, more preferably at least 1 week, 2 weeks, 3 weeks, most preferably at least 4 weeks,
f) optionally, detecting the labeled cell.

It is clear that the method also relates to a method of specifically labeling an epidermal stem cell in an epidermal culture.

Separately contacting a keratinocyte with the marker allows to label only cells derived from the keratinocyte. This separate labeling is advantageous as it provides for a more specific labeling of the epidermal stem cells (the epidermal stem cells present in an organotypic culture or skin equivalent shall be mainly or exclusively derived from keratinocytes which are used for seeding the culture).

From everything laid out in this specification it is clear that the invention also relates to a method of manufacturing an epidermal culture or kit according to the invention, comprising the steps of
a) providing a scaffold comprising esterified hyaluronic acid
b) introducing fibroblasts into the scaffold,
c) contacting a keratinocyte with a marker capable of labeling slowly proliferating cells,
d) adding the keratinocyte to the scaffold.

Preferably, the keratinocyte is contacted with the marker when adding the keratinocyte to the dermal portion of the skin equivalent of the present invention.

In another embodiment, the invention relates to a method for screening for markers capable of labeling, particularly specifically labeling, epidermal stem cells, comprising the steps of
a) providing an epidermal culture with labeled human epidermal stem cell according to any embodiment of the invention,
b) contacting the epidermal culture with a candidate for a marker capable of labeling, particularly specifically labeling epidermal stem cells,
c) comparing the pattern of labeling in the cell or culture according to step a) with the pattern of labeling in the cell or culture according to step b).

If the pattern of labeling between the markers according to a) and b) are different, then the candidate is considered not to be a marker capable of labeling, particularly specifically labeling epidermal stem cells. If the pattern of labeling observed in the cell(s) or culture according to step a) and step b) is the same or very similar, then the candidate for a marker is considered to be a marker capable of labeling, particularly specifically labeling, epidermal stem cells.

The candidate for a marker can be of any chemical structure as mentioned in the context of markers according to the invention.

Advantageously, the invention provides a gold standard for labeling of epidermal stem cells, which allows to perform screening for markers, particularly biochemical or molecular markers, capable of labeling epidermal stem cells. Thus, the present invention also allows to identify genes and/or gene products, which are specifically expressed or not expressed in epidermal stem cells.

In another embodiment, the invention also relates to a method of screening for agents (particularly cosmetics and/or therapeutics) capable of influencing epidermal stem cells, comprising the steps of
a) optionally providing a labeled human epidermal stem cell or an epidermal culture comprising such labeled human epidermal stem cell,
b) contacting the cell or culture with a candidate for an agent capable of influencing epidermal stem cells,
c) determining whether an epidermal stem cell according to a) or an epidermal stem cell in a culture according to a) is influenced by said agent.

If the epidermal stem cell is influenced by the candidate for an agent, then the candidate is confirmed as being an agent capable of influencing epidermal stem cells.

The influence can relate to any kind of behaviour of stem cells of interest, i.e. migration, proliferation, cell death (necrosis or apoptosis), shape change (e.g. flattening), or gene expression (e.g. expression of particular genes or gene products of interest, particularly of markers of differentiation, e.g. keratin K1, keratin K10, or filaggrin).

The agent can also be a candidate for a cosmetic or therapeutic agent, or it can be a candidate for the active ingredient in a cosmetic and/or therapeutic composition.

The invention also relates to a kit comprising a culture according to the invention. Advantageously, it has been found that the culture according to the invention can also be provided as a kit allowing to ship the culture to a different destination. Thus, it is possible to prepare cultures with labeled epidermal stem cells under standardized and reproduceable conditions and to ship such cultures to uses in different destinations. More particularly, a kit according to the invention comprises a culture in a suitable compartment, and, optionally, suitable media (and/or stock solutions for such media) and/or additives for further cultivating the culture. The kit may also include instructions for use, e.g. for further cultivating the culture.

The present invention also allows to isolate epidermal stem cells from an epidermal culture according to the invention, e.g. by dissociating the culture and isolating the cells by fluorescent cell sorting microdissection. Therefore, the present invention also relates to a method of manufacturing or obtaining isolated human epidermal stem cells. Furthermore, the invention thus relates to an isolated human epidermal stem cell or a cell culture comprising or consisting of isolated human epidermal stem cells.

It is evident that the present invention also relates to any uses of a cell, culture or kit according to the invention for screening of markers capable of labeling epidermal stem cells and/or for screening of agents capable of influencing epidermal stem cells and/or for studying epidermal development and/or epidermal stem cell behaviour and/or for isolating human epidermal stem cells.

All references cited in this description (including the examples) are herewith incorporated by reference with respect to their entire disclosure content and the disclosure content specifically mentioned in this specification.

The invention is further illustrated by the examples given below which are not intended to limit the scope of the invention in any way.

### Examples

### Example 1:

### Preparation of Dermal Equivalents (DEs) based on type I collagen hydrogels (col-DEs)

The preparation of DEs based on type I collagen hydrogels (col-DEs) was carried out as described previously in detail (Smola H, Stark HJ, Thiekotter G, Mirancea N, Krieg T, Fusenig NE (1998). Dynamics of basement membrane formation by keratinocyte-fibroblast interactions in organotypic skin culture. Exp Cell Res., vol. 239(2), pp. 399-410.; Stark HJ, Baur M, Breitkreutz D, Mirancea N, Fusenig NE (1999). Organotypic keratinocyte cocultures in defined medium with regular epidermal morphogenesis and differentiation. J Invest Dermatol., vol. 112(5), pp. 681-91). In brief, 80% (vol.) of a type I collagen in 0,1% acetic acid (4 mg per ml; rat tail tendon) were mixed on ice with 10% (vol.) of 10x Hanks' buffered saline containing phenol red and neutralized with 2 M NaOH. 10% (vol.) of a fibroblast suspension in FBS was added under stirring (final density 1 x 10⁵ cells per ml). This mixture was transferred into membrane filter inserts (Falcon no. 3901, 3-µm pores. BD-Biosciences, Heidelberg, Germany), placed in special deep well plates (BD-Bioscience no. 5467) and gelled at 37 °C. Glass rings fitting into the membrane inserts were placed on the gels for compression and to demarcate a central area for keratinocyte seeding. The gels were equilibrated for 24 h in FAD medium with 10% FBS, 10⁻¹⁰ M cholera toxin, 0,4 µg hydrocortisone and 50 µg L-ascorbic acid (Sigma) per ml; for further technical details see (Maas-Szabowski N., Stark, H.J., Fusenig, N.E. (2002). Cell interaction and epithelial differentiation, in: Freshney, R.I., Freshney, M. (eds.), Culture of epithelial cells. Wiley Liss, New York, pp. 31-63).

### Example 2:

### Co-cultures (skin equivalents, SEs) based on type I collagen hydrogel dermal equivalents (DEs)

Co-cultures on col-DEs (see Example 1) were started by seeding 1 x 10⁶ keratinocytes inside the glass rings onto the equilibrated collagen gels yielding a density of 3 x 10⁵ cells per cm². After submersed incubation overnight the glass rings were removed and the medium level was lowered to the base of the DEs, thus exposing the culture surface to the air-medium interface. Cultivation was continued in FAD medium with 10% FBS. 10⁻¹⁰ M cholera toxin, 0,4 µg hydrocortisone and 50 µg L-ascorbic acid per ml with medium change every other day.

### Example 3:

### Preparation of Dermal Equivalents (DEs) to be used as dermal portion of the skin equivalent based on hyaluronic acid scaffolds (hygraft-DEs)

Scaffold-based DEs were fabricated using Hyalograft-3D, a fleece-like non-woven fibrous material (about 1,2 mm thick) made of hyaluronic acid esterified with benzyl alcohol (FidiaAdvanced Biopolymers, Abano Terme, Italy). Human dermal fibroblasts were introduced into the scaffold as fibrin gel suspension. The fibrin gel was prepared using Baxter-Tissucol/Tisseel, a two-component fibrin glue for surgical application (Baxter, Heidelberg, Germany). The fibrinogen component was diluted with PBS without Ca²⁻ and Mg²⁺ pH 7.0 to a fibrinogen concentration of 8 mg per ml, while in the second component the original thrombin concentration was reduced to 10 units using PBS as diluent. Circular pieces of Hyalograft-3D with 22 mm diameter were placed into 4,5 cm² Falcon filter inserts (see above, Example 1) and overlaid with 600 µl of a 1:1 mixture of diluted thrombin and FBS containing 9 x 10⁵ fibroblasts. Immediately afterwards, 600 µl of fibrinogen dilution were added, thoroughly mixed and evenly distributed over the whole are of the scaffold by gentle pipetting. After about 10 min at 37 °C a clot enclosing the fibroblasts had formed, filing the space of the scaffold and forming a smooth surface. Finally, these constructs contained 2 x 10⁵ fibroblasts per cm², 4 mg fibrinogen and 2.5 units thrombin per ml. To prevent floating and to confine the central area for keratinocyte seeding, the constructs were covered by glass rings (as used for col-DEs, see Example 1) and submersed in DMEM medium with 10% FBS. 50 µg L-ascorbic acid and 1 ng rh TGFβ1 per ml. After pre-cultivation for one week in this medium (changes every other day) keratinocytes were seeded on top of the scaffolds (see Example 2).

### Example 4:

### Co-cultures (skin equivalents, SEs) based on hyaluronic acid scaffold dermal equivalents (hygraft-DEs)

The co-cultures on hygraft-DEs (hy-DEs) were generated as described in Example 2 with the exception that hygraft-DEs were used and that the the equilibration medium (before keratinocyte seeding) was supplemented with 500 units aprotinin (Bayer, Leverkusen, Germany) per ml which prevented precocious fibrinolysis by keratinocytes. At the time of keratinocyte seeding aprotinin was reduced to 200 units per ml and maintained at that level (medium changes every other day).

### Example 5:

### Labeling of epidermal stem cells using iododeoxyuridine

For IdU-labeling, keratinocytes are plated and labeled immediately for 12 hours by adding 1 mM IdU diluted froma 56 mM stock into the culture medium. After 12 hours maintenance, the medium is changed into a IdU-free medium. The procedure is repeated 4 to 6 times.

### Example 6:

### Labeling of epidermal stem cells using PKH26 or PKH67

Plated keratinocytes are trypsinized and counted for labeling with PKH26 (red fluorescent cell linker Mini kit (Sigma)) or PKH67 (green fluorescent cell linker minikit (Sigma)). The correct amounts of cells are washed twice in PBS and 4 x 10⁶ cells are diluted in 1 mm diluent. 6 ml PKH26 or 67 are diluted in the diluent and the cells and the fluorescent cell linker are mixed gently for 5 minutes. The labeling reaction is stopped by adding 1V 100 % FCS (2 ml). The cells are centrifuged and washed twice in PPS. The cells are resuspended in medium and counted. 1 x 10⁶ cells are plated for an organotypic keratinocyte co-culture as specified above. All steps of the staining protocol are carried out in the dark, if possible.

### Example 7:

### Obtaining keratinocytes and fibroblasts

Keratinocytes were isolated from adult human trunk skin, usually obtained from breast reduction surgery by a sequential treatment with thermolysin to separate the epidermis and with trypsin to release the cells according to Germain et al. (1993). Keratinocytes were plated at a density of 2 - 3 x 10⁴ cells per cm² on feeder cells (human dermal fibroblasts gamma-irradiated with 70 Gy, 1 x 10⁴ cells per cm²) in keratinocyte culture medium consisting of a 1:3-mixture of Ham's F12 and DMEM (traditionally named FAD medium (Wu YJ, Parker LM, Binder NE, Beckett MA, Sinard JH, Griffiths CT, Rheinwald JG. The mesothelial keratins: a new family of cytoskeletal proteins identified in cultured mesothelial cells and nonkeratinizing epithelia. Cell, vol. 31, pp. 693-703)) supplemented with 5 % FBS, 1,8 x 10⁻⁴ M adenine, 10⁻¹⁰ M cholera toxin, 0,4 µg hydrocortisone, 5 µg insulin (all Sigma, Deisenhofen, Germany) per ml. After one passage keratinocytes were cryo-preserved in aliquots of 2 x 10⁶ cells. Growth potential was tested in feeder layer based cloning assays which also excluded fibroblast contamination. The keratinocytes or thus obtained keratinocyte strains were used throughout Examples 1 to 6.

Human dermal fibroblasts were isolated from explant cultures of de-epidermised dermis. The fibroblasts were expanded up to passage 3-4 in DMEM with 10% FBS and cryo-preserved. The human dermal fibroblasts or thus obtained human dermal fibroblast strains were used throughout Examples 1 to 6.

## Claims

1. A skin equivalent comprising human epidermal stem cells which are specifically labeled with a marker capable of labeling slowly proliferating cells.

2. The skin equivalent according to claim 1, wherein the culture is an organotypic culture.

3. The skin equivalent according to claim 1 or 2, wherein the skin equivalent comprises a dermal portion.

4. The skin equivalent according to claim 3, wherein the skin equivalent is capable of surviving in culture for at least 2 weeks.

5. The skin equivalent according to any of claims 1 to 4, wherein the culture comprises a scaffold of fibrous material comprising esterified hyaluronic acid.

6. The skin equivalent according to any of claims 1 to 5, wherein the marker is a non-toxic maker and/or non-mutagenic marker.

7. The skin equivalent according to any of claims 1 to 6, wherein not more than 5% of the labeled cells are not human epidermal stem cells.

8. The skin equivalent according to any of claims 1 to 7, wherein at least 70% of the epidermal stem cells present in the culture are labeled.

9. A kit comprising a culture according to any of claims 1 to 8.

10. A method of manufacturing a skin equivalent of any one of claims 1 to 8 comprising the steps of
a) providing a scaffold comprising esterified hyaluronic acid
b) contacting a keratinocyte with a marker capable of labeling slowly proliferating cells,
c) introducing fibroblasts into the scaffold, and
d) adding the keratinocyte to the scaffold obtained by step c).

11. A skin equivalent obtainable by the method of claim 10.

12. Use of the skin equivalent of any one of claims 1 to 8 or claim 11 for screening of markers capable of labeling epidermal stem cells and/or for screening of agents capable of influencing epidermal cells and/or for studying of epidermal development and/or stem cell behavior and/or for isolating human epidermal stem cells.

13. A method for screening of markers capable of specifically labeling epidermal stem cells, comprising the steps of
a) providing an skin equivalent according to any of claims 1 to 8 or claim 11,
b) contacting the skin equivalent with a candidate for a marker capable of labeling, particularly specifically labeling epidermal stem cells,
c) comparing the pattern of labeling in the cell or culture according to step a) with the pattern of labeling in the cell or culture according to step b).

14. A method for screening of agents, particularly therapeutics or cosmetics, capable of influencing epidermal stem cells, comprising the steps of
a) contacting a culture according to any of claims 1 to 8 or claim 11 with a candidate for an agent capable of influencing epidermal stem cells,
b) determining whether the epidermal stem cell according to a) or an epidermal stem cell in a culture according to a) is influenced by the agent.
